Europäisches Patentamt

European Patent Office

Office européen des brevets

(11) Veröffentlichungsnummer: **0 503 441 A1**

## EUROPÄISCHE PATENTANMELDUNG

(21) Anmeldenummer: **92103596.0**

(22) Anmeldetag: **02.03.92**

(51) Int. Cl.5: **C09K 5/04**, C11D 7/50, C09K 3/30, C07C 17/42, C09K 15/02

(30) Priorität: **07.03.91 DE 4107245**

(43) Veröffentlichungstag der Anmeldung: **16.09.92 Patentblatt 92/38**

(84) Benannte Vertragsstaaten: **AT BE CH DE DK ES FR GB IT LI NL PT SE**

(71) Anmelder: **Solvay Fluor und Derivate GmbH Hans-Böckler-Allee 20 W-3000 Hannover 1(DE)**

(72) Erfinder: **Benecke, Thomas Erhardtstrasse 8 W-3000 Hannover 1(DE)**
Erfinder: **Zipfel, Lothar Ilseder Ring 11 W-3014 Laatzen 3(DE)**

(74) Vertreter: **Lauer, Dieter, Dr. c/o Solvay Deutschland GmbH, Postfach 220 W-3000 Hannover(DE)**

(54) Stabilisierung von Dichlortrifluorethan.

(57) Beschrieben wird ein Verfahren zur Stabilisierung von Dichlortrifluorethan enthaltenden Zusammensetzungen, bei dem man den Zusammensetzungen eine zur Stabilisierung des Dichlortrifluorethangehaltes wirksame Menge von mindestens einem Lithium- und/oder Erdalkalimetallsalz zusetzt oder die Zusammensetzungen damit in Kontakt bringt. Insbesondere Polyolvormischungen zur Herstellung von Polyurethan-Schaumstoffen, die Dichlortrifluorethan als Treibmittel sowie Polyole und aminbasische organische Substanzen und gegebenenfalls weitere übliche Zusatzstoffe vorformuliert enthalten, sind wirksam durch die Erfindung stabilisiert.

EP 0 503 441 A1

Die vorliegende Erfindung betrifft ein Verfahren zur Stabilisierung von Dichlortrifluorethan und Dichlortrifluorethan enthaltenden Zusammensetzungen, die Verwendung hierfür geeigneter Stabilisatoren sowie stabilisiertes Dichlortrifluorethan und stabilisierte Dichlortrifluorethan enthaltende Zusammensetzungen.

Dichlortrifluorethan kann als Ersatzstoff für die in ökologischer Hinsicht bedenklichen vollhalogenierten Fluorchlorkohlenwasserstoffe in vielen Anwendungsgebieten, z.B. als Reinigungs-, Spül- und Lösungsmittel, als Kältemittel und als Treibmittel in Aerosolen und zur Schaumkunststoffherstellung eingesetzt werden. Unter Dichlortrifluorethan versteht man dabei 1,1-Dichlor-2,2,2-trifluorethan ( = R123) und dessen isomere Verbindungen 1,2-Dichlor-1,1,2-trifluorethan ( = R123a) und 1,1-Dichlor-1,2,2-trifluorethan ( = R123b) oder auch deren Gemische. Da diese Stoffe naturgemäß über eine geringere Stabilität als die bisher eingesetzten vollhalogenierten Fluorchlorkohlenwasserstoffe verfügen, treten beim Einsatz dieser Ersatzstoffe in den genannten Anwendungsgebieten noch Stabilitätsprobleme auf. So konnte nachgewiesen werden, daß sich Dichlortrifluorethan, insbesondere R123, unter Bildung von Abbau- und/oder Umwandlungsprodukten zersetzen kann, was jedoch für die Anwendungen von Dichlortrifluorethan unerwünscht ist. Dieser Abbau und/oder diese Umwandlung von Dichlortrifluorethan führen z.B. zur Bildung anderer Halogenalkane wie beispielsweise R113 (Trichlortrifluorethan) oder R133a (1-Chlor-2,2,2-trifluorethan) aus Dichlortrifluorethan. Unter thermischer Belastung wird die Bildung dieser Abbau- und/oder Umwandlungsprodukte selbst in Dichlortrifluorethan ohne weitere Zusätze beobachtet. In Zusammensetzungen, die je nach Anwendung neben Dichlortrifluorethan noch weitere Bestandteile enthalten, wird sogar noch eine weit stärkere Bildung der erwähnten unerwünschten Abbau- und/oder Umwandlungsprodukte beobachtet. Besonders aminbasische Verbindungen, wie sie z.B. in vorformulierten Mischungen (z.B. aminhaltigen Polyolvormischungen) zur Herstellung von Schaumkunststoffen eingesetzt werden, können den unerwünschten Abbau bzw. die Umwandlung von Dichlortrifluorethan stark beschleunigen. Auch die Anwesenheit von Metall kann eine katalytische Wirkung auf die Umwandlung bzw. den Abbau von Dichlortrifluorethan ausüben. Dies ist besonders ungünstig, da Dichlortrifluorethan enthaltende Zusammensetzungen wie z.B. Kältemittel oder Treibmittelzusammensetzungen üblicherweise in Metallbehältern transportiert und gelagert werden.

Nachteilig wirken sich die beschriebenen Abbau- bzw. Umwandlungsvorgänge insbesondere bei der Verwendung von Dichlortrifluorethan als Treibmittel für die Herstellung von Polyurethan-Schaumstoffen aus. Polyurethan-Schaumstoffe werden erhalten, indem man Isocyanate mit mehrwertigen Alkoholen (Polyolen) in Gegenwart eines aminbasischen Katalysators und eines Treibmittels miteinander umsetzt. Hierfür werden zwei Komponenten miteinander vermischt, wobei die eine Komponente das Isocyanat ist, und die andere Komponente die Polyolvormischung ist, die das Polyol und aminbasische Verbindungen zusammen mit dem Treibmittel vorformuliert enthält. Durch die während der Polymerisation entstehende Reaktionswärme wird das Treibmittel verdampft und so ein geschäumtes Produkt von geringer Dichte erhalten. Da die Polyolvormischung neben dem Treibmittel einen hohen Anteil an aminbasischen organischen Verbindungen wie Aminen, Aminoalkoholen und/oder Aminogruppen enthaltenden Polyolen enthält, kann bei Verwendung von Dichlortrifluorethan als Treibmittel in den erwähnten Polyolvormischungen der unerwünschte Ab- bzw. Umbau von Dichlortrifluorethan durch die katalytische Einwirkung der aminbasischen Bestandteile in einem unerwünscht hohen Ausmaß stattfinden. Bei der üblichen Aufbewahrung der Polyolvormischungen in Stahlblechbehältern oder Metallfässern wird die maximale Lagerzeit dieser Polyolvormischungen durch die zusätzliche katalytische Einwirkung der Metalle weiter herabgesetzt.

Es ist bekannt, organische Stabilisatorsubstanzen, wie z.B. Alloocimen, 3-Methylbuten-3-ol-1, Nitromethan, dimeres α-Methylstyrol, α-Methylstyrol, Styrol oder Anethol zur Stabilisierung von Chlorfluoralkan enthaltenden Polyolvormischungen zu verwenden, um damit einige der Alterungserscheinungen zu unterdrücken. Diese Stabilisatoren können zwar die vom Einsatz von herkömmlichen Fluorchlorkohlenwasserstoffen bekannten Alterunserscheinungen wie zunehmende Startzeiten, verlängerte Abbindzeiten und verlängerte Klebefreizeiten positiv beeinflußen, aber nur ungenügend oder nur in hohen Konzentrationen die Bildung unerwünschter Abbau- und Umwandlungsprodukte aus Dichlortrifluorethan unterdrücken. Wird eine gewisse Lagerzeit überschritten, so wird auch bei den mit den bisherigen organischen Stabilisatoren stabilisierten Dichlortrifluorethan enthaltenden Polyolvormischungen eine Zersetzung des Dichlortrifluorethan in unerwünscht hohem Ausmaß festgestellt.

Es bestand daher die Aufgabe, ein Verfahren zur Stabilisierung von Dichlortrifluorethan gegen Zersetzung unter Bildung von Abbau- und/oder Umwandlungsprodukten bereitzustellen und für die Verwendung zur Stabilisierung von Dichlortrifluorethan geeignete Substanzen anzugeben. Weiterhin bestand die Aufgabe, derartig stabilisiertes Dichlortrifluorethan und stabilisierte Dichlortrifluorethan enthaltende Zusammensetzungen für verschiedene Anwendungsgebiete zur Verfügung zu stellen.

Überraschenderweise wurde nun gefunden, daß Dichlortrifluorethan stabilisiert werden kann, indem man Dichlortrifluorethan Lithium- und/oder Erdalkalimetallsalze zusetzt.

Gegenstand der Erfindung ist daher ein Verfahren zur Stabilisierung von flüssigen Dichlortrifluorethan enthaltenden Zusammensetzungen, dadurch gekennzeichnet, daß man den Zusammensetzungen eine zur Stabilisierung des Dichlortrifluorgehaltes wirksame Menge von mindestens einem Lithium- und/oder Erdalkalimetallsalz zusetzt oder die Zusammensetzungen damit in Kontakt bringt.

Zweckmäßige Erdalkalimetallsalze im Sinne der Erfindung sind insbesondere die Salze der Erdalkalimetalle der 3. bis 6. Periode (Mg, Ca, Sr, Ba), besonders die der 3. bis 5. Periode (Mg, Ca, Sr). Ganz besonders bevorzugt für das erfindungsgemäße Verfahren sind die Salze der Erdalkalimetalle Magnesium und Calcium.

Das Lithium- und/oder Erdalkalimetallsalz kann beispielsweise ein anorganisches Salz wie z.B. ein Nitrat oder Halogenid wie Bromid oder Chlorid sein. Auch ein organisches Salz einer niederen organischen Carbonsäure, insbesondere einer $C_1$- bis $C_4$-Carbonsäure, mit dem Lithium- und/oder Erdalkalimetall ist möglich. Vorzugsweise werden die Salze, die wasserfrei oder hydrathaltig sein können, als Feststoffe zugefügt. Die Salze können gegebenenfalls auch als wäßrige, dann aber möglichst konzentrierte Lösungen, z.B. als gesättigte Lösungen, zur Stabilisierung von Dichlortrifluorethan eingesetzt werden. Insbesondere bei der Stabilisierung von Polyolvormischungen zur Polyurethanverschäumung, die Dichlortrifluorethan als Treibmittel enthalten, sollten die obigen Salze möglichst wasserfrei oder in nur wenig freiem Wasser gelöst verwendet werden. Bevorzugt erfolgt die Stabilisierung von Polyolvormischungen, die Dichlortrifluorethan als Treibmittel enthalten, durch Zusatz fester Lithium- und/oder Erdalkalisalze.

In einer besonders bevorzugten Ausführung des erfindungsgemäßen Verfahrens setzt man als Lithium- und/oder Erdalkalimetallsalz ein Chlorid oder Nitrat ein. Vorteilhaft sind insbesondere die Chloride der Erdalkalimetalle Magnesium und/oder Calcium. Bevorzugt werden diese Salze als Feststoffe im erfindungsgemäßen Verfahren zur Stabilisierung eingesetzt. Beispiele für geeignete Salze sind $CaCl_2$, $CaCl_2 \cdot 2H_2O$, $CaCl_2 \cdot 4H_2O$, $CaCl_2 \cdot 6H_2O$, $MgCl_2$, $MgCl_2 \cdot 2H_2O$, $MgCl_2 \cdot 4H_2O$, $MgCl_2 \cdot 6H_2O$.

Im erfindungsgemäßen Verfahren werden den Dichlortrifluorethan enthaltenden Zusammensetzungen die Lithium- und/oder Erdalkalimetallsalze in einem Mengenbereich von 0,05 bis 5 Gew.-%, vorzugsweise von 0,5 bis 2 Gew.-%, bezogen auf das in den Zusammensetzungen enthaltende Dichlortrifluorethan als 100 Gew.-%, zugefügt.

Das erfindungsgemäße Verfahren eignet sich nicht nur zur Stabilisierung von Dichlortrifluorethan allein, sondern in vorteilhafter Weise auch zur Stabilisierung von Zusammensetzungen, die Dichlortrifluorethan zusammen mit weiteren Bestandteilen enthalten. Die Bildung von Abbau- und/oder Umwandlungsprodukten in Dichlortrifluorethan allein bzw. in Dichlortrifluorethan enthaltenden Zusammensetzungen kann somit wirksam unterdrückt werden. Dichlortrifluorethan im Sinne der Erfindung umfaßt hierbei die Isomeren 1,1-Dichlor-2,2,2-trifluorethan (= R123), 1,2-Dichlor-1,1,2-trifluorethan (= R123a) und 1,2-Dichlor-1,2,2-trifluorethan (= R123b) sowie die Isomeren-Gemische. Das Verfahren eignet sich zur Stabilisierung von Dichlortrifluorethan und Dichlortrifluorethan enthaltenden Zusammensetzungen für vielfältige Anwendungsgebiete, z.B. bei Verwendung von Dichlortrifluorethan in Reinigungs- und Lösungsmitteln, in Trennmitteln, in Kältemitteln und in Treibmitteln. Vorzugsweise ist das erfindungsgemäße Verfahren zur Stabilisierung von Kältemittel- oder Treibmittelzusammensetzungen geeignet. Besonders eignet sich das erfindungsgemäße Verfahren zur Stabilisierung von Treibmittelzusammensetzungen, die zur Herstellung von Schaumkunststoffen, beispielsweise bei der Herstellung von Polyolefin-Schaumstoffen und bei der Herstellung von Polyurethan-Schaumstoffen, eingesetzt werden. Es können auch aminhaltige Polyolvormischungen, welche Dichlortrifluorethan und aminbasische organische Substanzen wie z.B. Gemische aus Aminen und Polyolen und/oder Aminogruppen enthaltende Polyole und gegebenenfalls weitere an sich übliche Zusätze enthalten, und die zur Herstellung von Polyurethan-Schaumstoffen dienen, wirksam mit dem erfindungsgemäßen Verfahren stabilisiert und die Bildung von Abbau- und Umwandlungsprodukten aus Dichlortrifluorethan unterdrückt werden.

Die Erfindung betrifft ebenfalls stabilisierte Zusammensetzungen, die Dichlortrifluorethan und eine stabilisierend wirksame Menge von mindestens einem Lithium- und/oder Erdalkalimetallsalz enthalten. In den erfindungsgemäßen Dichlortrifluorethan enthaltenden Zusammensetzungen liegen die eingesetzten Lithium- und/oder Erdalkalimetallsalze entweder suspendiert, gelöst oder als Lösung mit Bodenkörper vor. Im einfachsten Fall bestehen diese Zusammensetzungen aus Dichlortrifluorethan selbst, welches eine stabilisierend wirksame Menge eines Lithium- und/oder Erdalkalimetallsalzes enthält. Auch umfaßt die Erfindung stabilisierte Dichlortrifluorethan enthaltende Zusammensetzungen, die noch weitere für den jeweiligen Anwendungszweck übliche Bestandteile enthalten. Solche Bestandteile können beispielsweise weitere organische Lösungsmittel, Treibmittel und auch oberflächenaktive Substanzen sein. Beispiele für weitere Lösungsmittel sind unter anderem niedere Alkohole, cyclische oder offenkettige, gesättigte oder ungesättigte Ketone mit 3 bis 9 Kohlenstoffatomen, cyclische oder offenkettige Ether mit 2 bis 5 Kohlenstoffatomen, oder Niederalkylester von Monocarbonsäuren oder Dicarbonsäuren mit bis zu 18 Kohlenstoffatomen, insbesondere vom niederen Carbonsäuren, cyclische oder offenkettige Kohlenwasserstoffe oder flüssige Paraffine. Beispiele für oberflächenaktive Substanzen sind unter anderem höhere Alkohole mit bis zu 17 Kohlenstoffatomen.

Erfindungsgemäße Dichlortrifluorethan enthaltende Zusammensetzungen können beispielsweise als Kältemittel, Lösungsmittel, Trennmittel oder Treibmittel verwendet werden. Ebenfalls als Hilfsmittel in der Metallbearbeitung, z.B. beim Schneiden oder Bohren von Metall, können Dichlortrifluorethan enthaltende Zusammensetzungen, z.B. Mischungen aus Dichlortrifluorethan mit für Schneidmittel üblichen Zusatzstoffen wie Alkoholen, Ethern oder Estern langkettiger Carbonsäuren verwendet werden. Insbesondere eignen sich erfindungsgemäße Zusammensetzungen als Treibmittelzusammensetzungen, z.B. zur Aerosolversprühung oder in der Schaumstoffherstellung, und als Kältemittelzusammensetzungen, in denen erfindungsgemäß stabilisiertes Dichlortrifluorethan allein oder im Gemisch mit weiteren Bestandteilen enthalten ist. Dichlortrifluorethan enthaltende Kältemittel können beispielsweise dadurch erfindungsgemäß stabilisiert sein, daß sie mit festem Calciumchlorid und/oder Magnesiumchlorid in Kontakt gebracht werden. In einer Variante der Erfindung können so z.B. Dichlortrifluorethan enthaltende Kältemittel im Kühlkreislauf über festes Calciumchlorid und/oder Magnesiumchlorid geleitet werden und somit die Bildung unerwünschter Abbau- und Umlagerungsprodukte aus Dichlortrifluorethan unterdrückt werden.

Bevorzugt umfaßt die Erfindung stabilisierte Treibmittelzusammensetzungen, die zur Herstellung von Schaumkunststoffen wie Polyolefin-Schaumstoffen oder Polyurethan-Schaumstoffen eingesetzt werden.

In einer besonders bevorzugten Variante betrifft die Erfindung stabilisierte Polyolvormischungen für die Herstellung von Polyurethan-Schaumstoffen, enthaltend Dichlortrifluorethan und monomere organische Amine und Polyole und/oder Aminogruppen enthaltende Polyole und gegebenenfalls weitere an sich übliche Zusätze. Derartige Polyolvormischungen, die als Treibmittel Dichlortrifluorethan allein oder ein Treibmittelgemisch aus Dichlortrifluorethan und anderen flüssigen oder gasförmigen Treibmitteln, z.B. Treibgasen wie Kohlendioxid, enthalten können, sind erfindungsgemäß durch den Zusatz von mindestens einem Lithium- und/oder Erdalkalimetallsalz in Mengenbereichen von 0,05 bis 5 Gew.-%, vorzugsweise 0,5 bis 2 Gew.-%, bezogen auf Dichlortrifluorethan als 100 Gew.-%, stabilisiert. Insbesondere enthalten die erfindungsgemäßen Polyolvormischungen Calciumchlorid und/oder Magnesiumchlorid. Als basische Substanzen können monomere organische Amine und/oder Aminogruppen enthaltende Polyole enthalten sein. Die in den erfindungsgemäßen Polyolvormischungen enthaltenen monomeren organischen Amine können offenkettige oder cyclische, tertiäre oder sekundäre, gesättigte oder ungesättigte Amine sein, welche als Starter der Polyadditionsreaktion dienen können, beispielsweise durch niederes Alkyl oder Hydroxyalkyl substituierte Alkylamine, Alkylendiamine, Alkanolamine, Guanidine und cyclische Amine wie Piperidine, Pyridine oder Morpholine. So können in den erfindungsgemäßen Polyolvormischungen an sich bekannte gesättigte oder ungesättigte aliphatische, cycloaliphatische oder heterocyclische Amine, insbesondere z.B. Triethylamin, Methyl-bis-dimethylaminoethyl-amin, N,N,N',N'-Tetramethylethylendiamin, Triethylendiamin, Dimethylcyclohexylamin, N,N,N',N'-Tetramethyl-1,3-butandiamin, 1,1,3,3-Tetramethylguanidin, 1,2,4-Trimethylpiperazin, N-Cyclohexylpiperidin, 4-Dimethylaminopyridin, N-Methylmorpholin oder Morpholin, eingesetzt werden. Auch andere übliche Amine, wie z.B. N-Ethylmorpholin, N,N'-Dimethylbenzylamin, N,N-Dimethyl-(N,N'-Dimethylamino)-piperazin, N,N-Dimethylpiperazin, Hexyldecyldimethylamin, Diethylcyclohexylamin, N-Phenylcyclohexylamin sind möglich. Weiterhin können als Amine an sich bekannte Aminoalkohole, insbesondere z.B. Dimethylethanolamin, Diethanolamin, N-Methyldiethanolamin, Triethanolamin, Diisopropanolamin oder N,N,N',N'-Tetrakis(2-hydroxypropyl)ethylendiamin, eingesetzt werden. Selbstverständlich umfassen die erfindungsgemäßen Polyolvormischungen auch Dichlortrifluorethan-haltige Polyolvormischungen mit anderen üblichen Aminoalkoholen, wie z.B. Diethylethanolamin oder 1,4-Bis-(2-hydroxypropyl)-2-methylpiperazin. Auch Aminogruppen-haltige Polyole, z.B. mit Aminen, Alkanolaminen oder aromatischen Aminen gestartete Polyether mit Restgehalt an Hydroxylgruppen, können in den erfindungsgemäßen Polyolvormischungen enthalten sein. Solche Polyole auf der Grundlage von Aminen sind z.B. Reaktionsprodukte von Ethylen- und bzw. oder Propylenoxid mit Aminen wie z.B. Diethylentriamin, Ethylendiamin, Triethanolamin oder Toluylendiamin. Es versteht sich, daß im Sinne der Erfindung auch andere als die genannten, üblicherweise verwendeten Polyolsysteme eingesetzt werden können. In einer Variante wird z.B. ein aminbasisches Polyol mit einer Hydroxylzahl von 480 bis 510 eingesetzt.

Auch weitere Zusätze, z.B. Zusätze zur Schaumstabilisierung wie z.B. Polyethersiloxan-Copolymere oder Gemische aus organischen Tensiden und Polyethersiloxan-Copolymeren, können in den erfindungsgemäßen Polyolvormischungen enthalten sein. Als weiterer Zusätze können auch an sich bekannte Additive, z.B. Flammschutzmittel oder Farbstoffe enthalten sein. Diese Zusätze können in den erfindungsgemäßen Polyolvormischungen in den an sich für diese Zusätze üblichen Mengen vorliegen.

In einer erfindungsgemäßen Polyolvormischung können so beispielsweise, bezogen auf 100 Gewichtsteile eines aminbasischen Polyols mit einer Hydroxylzahl von z.B. 485, 2 bis 5 Gewichtsteile Dimethylcyclohexylamin, 0,5 bis 2 Gewichtsteile Polysiloxan, gegebenenfalls 0,5 bis 3,5 Gewichtsteile Wasser mit bis zu 40, insbesondere 10 bis 40, Gewichtsteile Dichlortrifluorethan als Treibmittel sowie 0,05 bis 5 Gew.-%-$CaCl_2$, bezogen auf die in der Polyolvormischung enthaltenden Dichlortrifluorethanmenge, enthalten sein.

Den erfindungsgemäßen Polyolvormischungen können zusätzlich noch weitere an sich übliche Zusätze, insbesondere im Stand der Technik an sich bekannte Stabilisatoren wie z.B. dimeres $\alpha$-Methylstyrol, 3-Methylbuten-3-ol-1, Alloocimen, Butadien, Isopren, Styrol, Anethol, m-Diisopropenylbenzol, $\alpha$-Methylstyrol, 1,3,5-Triisopropenylbenzol, 1-(p-Methoxyphenyl)-2-nitro-propen-1, Nitromethan oder p-Isopropenyltoluol, etc. in den üblichen Mengen zugesetzt sein.

Durch die Erfindung wird ein überraschend einfaches und effektives Verfahren bereitgestellt, um in Dichlortrifluorethan enthaltenden Zusammensetzungen die Bildung von Abbau- und/oder Umlagerungsprodukten zu unterdrücken. Erfindungsgemäß werden stabilisiertes Dichlortrifluorethan und stabilisierte Dichlortrifluorethan enthaltende Zusammensetzungen mit vorteilhaften Eigenschaften zur Verfügung gestellt. Das erfindungsgemäß stabilisierte Dichlortrifluorethan und die erfindungsgemäß stabilisierten Zusammensetzungen sind über lange Zeiträume stabil, d.h. die Bildung von Abbau- und/oder Umwandlungsprodukten aus Dichlortrifluorethan wird wirksam unterdrückt. Dies gilt selbst bei Gegenwart von Kesselstahl, so daß die erfindungsgemäße Stabilisierung auch bei der üblichen Lagerung von Dichlortrifluorethan bzw. Dichlortrifluorethan enthaltenden Zusammensetzungen wie Kältemitteln oder Polyolvormischungen in Stahlblechbehältern oder Metallfässern besonders geeignet ist. So übertreffen insbesondere die erfindungsgemäß stabilisierten Polyolvormischungen nicht erfindungsgemäß stabilisierte, mit Dichlortrifluorethan als Treibmittel versehene Polyolvormischungen in hervorragender Weise in ihrer Stabilität und guten Lagerfähigkeit.

Auch im fertigen Schaumstoff, der mit Hilfe von erfindungsgemäß stabilisiertem Dichlortrifluorethan als Treibmittel hergestellt wurde, wird eine deutlich geringere Zersetzung von Dichlortrifluorethan festgestellt. Beispielsweise kann so bei aus den erfindungsgemäßen Polyolvormischungen hergestellten Polyurethanschaumstoffen, bei denen das Treibmittel Dichlortrifluorethan in den geschlossenen Schaumstoffporen verbleibt, eine gute Stabilisierung von Dichlortrifluorethan erreicht werden. Hier zeigen sich die erfindungsgemäß eingesetzten Lithium- und/oder Erdalkalimetallsalze, insbesondere Magnesiumchlorid und Calciumchlorid, den bekannten organischen Stabilisatorsubstanzen überlegen, da sie Dichlortrifluorethan sowohl in der Polyolvormischung als auch im fertigen Schaumstoff stabilisieren können. Die unerwünschte Zersetzung von Dichlortrifluorethan im fertigen Schaumstoff läßt sich selbstverständlich auch dadurch unterdrücken, daß man die erfindungsgemäß eingesetzten Lithium- und/oder Erdalkalimetallsalze kombiniert mit den bekannten organischen Stabilisatorsubstanzen, z.B. Alloocimen, dimeres $\alpha$-Methylstyrol, 3-Methylbuten-3-ol-1 oder Nitromethan, die auf Dichlortrifluorethan eine stabilisierende Wirkung im Schaumstoff haben.

Die erfindungsgemäßen Polyolvormischungen eignen sich somit sehr gut für die Herstellung von Schaumstoffen hoher Qualität, die z.B. als Konstruktionswerkstoff oder als Wärmedämmstoff bzw. als Kälteisoliermaterial im Wohn- oder Lebensmittelbereich eingesetzt werden können.

Ein weiterer Vorteil des erfindungsgemäß stabilisierten Dichlortrifluorethan und der erfindungsgemäß stabilisierten Dichlortrifluorethan enthaltenden Zusammensetzungen besteht in ihrer günstigen Temperaturstabilität über einen weiten Temperaturbereich. Beispielsweise läßt die stabilisierende Wirkung der erfindungsgemäß verwendeten Lithium- und/oder Erdalkalimetallsalze selbst bei Temperaturen bis zu 110 °C kaum nach.

Die nachfolgenden Beispiele sollen die Erfindung näher erläutern, ohne sie jedoch in ihrem Umfang zu begrenzen.

Beispiele

A) Stabilisierung von Dichlortrifluorethan in Polyolvormischungen:

Der Nachweis der erfindungsgemäßen Stabilisierung gegen die Zersetzung von Dichlortrifluorethan erfolgt in den nachfolgenden Beispielen 1 - 15 in Polyolvormischungen zur Polyurethan-Schaumstoffherstellung, in denen ohne erfindungsgemäße Stabilisierung die Zersetzung von Dichlortrifluorethan unter Bildung von Abbau- und/oder Umwandlungsprodukten durch die Gegenwart basischer organischer Substanzen besonders stark katalysiert wird.
Es wurde eine Polyolvormischung mit folgenden typischen Bestandteilen verwendet:

| 100 Gewichtsteile | Aminogruppenhaltiger Polyether auf Aminbasis mit einer Hydroxylzahl von 485 |
| 3 Gewichtsteile | Dimethylcyclohexylamin |
| 1 Gewichtsteil | Polysiloxan (Si-Copolymer) |
| 35 Gewichtsteile | Dichlortrifluorethan |

Je einer Probe dieser Polyolvormischung wurde eine wirksame Menge eines erfindungsgemäß verwendeten Lithium- oder Erdalkalimetallsalzes zugegeben und die Probe in einem Glasgefäß bei 54 °C für 4 Wochen bis 7 Wochen gelagert. Durch die Lagerung bei einer gegenüber Raumtemperatur höheren Temperatur konnten die Stabilisierungsversuche in erheblich kürzerer Versuchszeit als bei Raumtemperatur durchgeführt werden: 6 Wochen Lagerung bei 54 °C entsprechen etwa einem halben Jahr Lagerzeit bei Raumtemperatur.

Nach Lagerung wurden die Zusammensetzungen auf die Bildung von Abbau- und Umwandlungsprodukten aus Dichlortrifluorethan geprüft. Hierfür wurden aus der Gasphase über den Zusammensetzungen mit einer Einwegspritze Proben mit einer definierten Volumenmenge (1 ml) entnommen, die gaschromatographisch untersucht wurden. Es wurde hierfür ein Gaschromotograph mit Wärmeleitfähigkeitsdetektor (Modell Hewlett-Packard 5890 II, Haysept Q-Säule)eingesetzt. Für während der Lagerung aus Dichlortrifluorethan durch Zersetzung gebildete Abbau- und Umwandlungsprodukte wurde stellvertretend auf die Anwesenheit von 1-Chlor-2,2,2-trifluorethan (= R133a) geprüft, da R133a sich analytisch sowohl qualitativ als auch quantitativ leicht erfassen läßt. Die jeweilige Menge an R 133a in den Proben wurde anhand von Referenz-Proben, die eine definierte Menge an R 133a mit Luft verdünnt enthielten, ermittelt. Die untere Nachweisgrenze lag bei 5 ppm.

Die nachfolgende Tabelle I zeigt die erhaltenen Ergebnisse. Die angegebenen Konzentrationen der verwendeten Stabilisatorsubstanzen beziehen sich auf das in den Zusammensetzungen enthaltende Dichlortrifluorethan als 100 Gew.-%. Die Salze wurden als solche oder in wäßrigen gesättigten Lösungen eingesetzt.

Tabelle I

| Erfindungsgemäße Stabilisierung von Dichlortrifluorethan in Polyolvormischungen | | | | |
|---|---|---|---|---|
| Bsp. Nr. | Stabilisierung mit | Konzentration*) [Gew.-%] | Lagerung bei 54 °C [Wochen] | Menge an R133a in der Gasphase [ppm] |
| 1 | SrCl$_2$ | 5 | 4 | <5 |
| 2 | CaCl$_2$·2H$_2$O | 0,5 | 7 | <5 |
| 3 | CaCl$_2$·2H$_2$O | 1 | 7 | <5 |
| 4 | CaCl$_2$·2H$_2$O | 2 | 7 | <5 |
| 5 | CaCl$_2$·2H$_2$O | 5 | 7 | <5 |
| 6 | CaCl$_2$ | 1 | 6 | <5 |
| 7 | MgCl$_2$·6H$_2$O | 0,5 | 6 | <5 |
| 8 | MgCl$_2$·6H$_2$O | 1 | 6 | <5 |
| 9 | MgCl$_2$·6H$_2$O | 2 | 6 | <5 |
| 10 | MgCl$_2$·6H$_2$O | 5 | 6 | <5 |
| 11 | Ca(NO$_3$)$_2$-Lösung (ges.) | 1 | 6 | <5 |
| 12 | Ca(NO$_3$)$_2$-Lösung (ges.) | 2 | 6 | <5 |
| 13 | Ca(NO$_3$)$_2$-Lösung (ges.) | 5 | 6 | <5 |
| 14 | LiCl-Lösung (ges.) | 2 | 4 | <5 |
| 15 | LiCl-Lösung (ges.) | 5 | 6 | <5 |

*) bezogen auf Dichlortrifluorethan
untere Nachweisgrenze: 5ppm ges.: gesättigte Lösung

Die Ergebnisse belegen die hervorragende stabilisierende Wirkung der erfindungsgemäß verwendeten Lithium- und Erdalkalimetallverbindungen. Die erfindungsgemäßen Polyolvormischungen mit Dichlortrifluorethan als Treibmittel sind wirksam gegen die unerwünschte Bildung von Abbau- und Umwandlungsprodukte aus Dichlortrifluorethan stabilisiert. Damit besitzen die erfindungsgemäßen Polyolvormischungen eine sehr gute Lagerstabilität.

Vergleichsbeispiele 1 und 2:

Zum Vergleich wurden Polyolvormischungen mit den oben angegebenen Bestandteilen ohne erfindungsgemäßer Stabilisierung unter ansonsten analogen Bedingungen nach Lagerzeiten von 1 und 6 Wochen ebenfalls auf die Bildung von R133a untersucht. Die Analyse erfolgte, wie bereits beschrieben. Hier bildeten sich bereits nach 1 Woche Lagerung bei 54 °C erhebliche Mengen an R133a:

| Vergleichsbeispiel | Lagerung der Polyolvormischung ohne erfindungsgemäße Stabilisierung bei 54 °C | Menge an R133a in der Gasphase |
|---|---|---|
| V1 | 1 Woche | 390 ppm |
| V2 | 6 Wochen | 1190 ppm |

Im Gegensatz zu den erfindungsgemäßen Polyolvormischungen werden in Polyolvormischungen mit Dichlortrifluorethan als Treibmittel nach dem bisherigen Stand der Technik erhebliche Mengen an Abbau- und Umwandlungsprodukten aus Dichlortrifluorethan beobachtet. Die Lagerstabilität ist damit im Vergleich zu den erfindungsgemäßen Polyolvormischungen deutlich geringer.

B) Stabilisierung von Dichlortrifluorethan in Polyurethan-Schaumstoffen:

In den nachfolgenden Beispielen 16 - 19 erfolgt der Nachweis der erfindungsgemäßen Stabilisierung gegen die Zersetzung von Dichlortrifluorethan in geschlossenzelligen Polyurethan-Hartschaumstoffen.
Der Polyurethan-Hartschaumstoff wurde durch Umsetzung einer Dichlortrifluorethan enthaltenden Polyolvormischung mit Diisocyanat MDI 110 (Diphenylmethan-4,4'-diisocyanat) in einer üblichen Verschäumungsapparatur bei Raumtemperatur hergestellt. Die verwendete Polyolvormischung bestand aus den folgenden Bestandteilen:

| 100 Gewichtsteile | Aminogruppenhaltige Polyether auf Aminbasis mit einer Hydroxylzahl von 485 |
|---|---|
| 3 Gewichtsteile | Dimethylcyclohexylamin |
| 1 Gewichtsteil | Polysiloxan (Si-Copolymer) |
| 0,5 Gewichtsteile | Wasser |
| 35 Gewichtsteile | Dichlortrifluorethan |

Es wurden einer Probe dieser Polyolvormischung zusätzlich noch 1,75 Gewichtsteile eines erfindungsgemäßen stabilisierenden Salzes zugefügt, was einer Menge von 5 Gew.-%, bezogen auf das in den Zusammensetzungen enthaltende Dichlortrifluorethan, entspricht. Zum Vergleich wurden für die Polyurethan-Schaumstoffherstellung auch eine Probe der Polyolvormischung ohne Stabilisatorzusatz eingesetzt.
Den Polyolvormischungen wurde in für die Herstellung von Polyurethan-Schaumstoffen üblichen Weise 140 Gewichtsteile Diisocyanat MDI 110 (Diphenylmethan-4,4'-diisocyanat) zugemischt, wodurch ein geschlossenzelliger Polyurethan-Hartschaumstoff erhalten wurde. Die erhaltenen Polyurethan-Hartschaumstoffe wurden anschließend bei 20 °C oder bei 54 °C für 3 bis 14 Tage gelagert. Nach Lagerung wurden die Polyurethan-Hartschaumstoffe auf die Bildung von Abbau- und Umwandlungsprodukten aus Dichlortrifluorethan geprüft. Hierfür wurde den Polyurethan-Hartschaumstoffen aus den Schaumstoffporen mit einer Einwegspritze eine definierte Volumenmenge Gas (1 ml) entnommen, die gaschromatographisch untersucht wurde. Wie bereits unter A) für die Polyolvormischungen beschrieben, wurde für während der Lagerung aus Dichlortrifluorethan durch Zersetzung gebildete Abbau- und Umwandlungsprodukte stellvertretend auf die Anwesenheit von 1-Chlor-2,2,2-trifluorethan (= R133a) geprüft. Für diese Untersuchungen wurde ein Gaschromatograph mit Wärmeleitfähigkeitsdetektor (Modell HP 5890 II, Haysept Q-Säule) eingesetzt, der, wie ebenfalls bereits unter A) beschrieben, zuvor mit definierten Mengen an R133a geeicht worden war.
Der nachfolgenden Tabelle II können die erhaltenen Ergebnisse nach Lagerung der Polyurethan-Hartschaumstoffe bei 20 °C entnommen werden. Tabelle III zeigt die Ergebnisse, die nach Lagerung der Polyurethan-Hartschaumstoffe bei 54 °C erhalten wurden. Zum Vergleich sind in beiden Tabellen auch die erhaltenen Analysenergebnisse von Schäumen aufgeführt, die aus Dichlortrifluorethan enthaltenden Polyolvormischungen ohne weiteren Stabilisatorzusatz hergestellt wurden (Vergleichsbeispiele 3 und 4).

Tabelle II

| Stabilisierung von Dichlortrifluorethan in Polyurethan-Schaumstoffen mit 5 Gew.-% Stabilisator (bezogen auf Dichlortrifluorethan) nach Lagerung bei 20 °C | | | | | |
|---|---|---|---|---|---|
| Bsp. Nr. | Stabilisierung mit | Menge an R133a im Schaumstoff [ppm] nach | | | |
| | | 3 Tagen | 5 Tagen | 10 Tagen | 14 Tagen |
| 16 | $CaCl_2 \cdot 2H_2O$ | 239 | 279 | 302 | 342 |
| V3 | ohne Zusatz | 302 | 369 | 375 | 439 |

Tabelle III

| Stabilisierung von Dichlortrifluorethan in Polyurethan-Schaumstoffen mit 5 Gew.-% Stabilisator (bezogen auf Dichlortrifluorethan) nach Lagerung bei 54 °C | | | | | |
|---|---|---|---|---|---|
| Bsp. Nr. | Stabilisierung mit | Menge an R133a im Schaumstoff [ppm] nach | | | |
| | | 3 Tagen | 5 Tagen | 10 Tagen | 14 Tagen |
| 17 | $CaCl_2 \cdot 2H_2O$ | 140 | 310 | 374 | 501 |
| V4 | ohne Zusatz | 275 | 390 | 507 | 764 |

Die Ergebnisse zeigen, daß in Polyurethan-Schaumstoffen, die mit erfindungsgemäß stabilisierten Polyolvormischungen hergestellt wurden, weniger unerwünschte Abbau- und Umbauprodukte aus Dichlortrifluorethan, wie z.B. R133a, gebildet werden. Die erfindungsgemäße Stabilisierung von Dichlortrifluorethan durch Calciumchlorid bleibt auch auf länger Zeit erhalten. Dies zeigt sich insbesondere bei der Lagerung bei 54 °C, wo auch nach Ablauf von 14 Tagen die erfindungsgemäß mit Calciumchlorid stabilisierten Polyurethan-Schaumstoffe deutlich geringere Mengen an R133a im Vergleich zu den ohne Stabilisatorzusatz hergestellten Polyurethan-Schaumstoffen aufweisen. Aus Tabelle III geht auch hervor, daß die erfindungsgemäße Stabilisierung von Dichlortrifluorethan durch Calciumchlorid auch in höheren Temperaturbereichen wirksam ist.

Zusätzlich wurde auch die Wirksamkeit der erfindungsgemäßen Stabilisierung von Dichlortrifluorethan in Polyurethan-Schaumstoffen in Kombination mit den bekannten organischen Stabilisatorsubstanzen überprüft. Vor der Verschäumung wurden hierfür den Dichlortrifluorethan enthaltenen Polyolvormischungen jeweils ein binäres Stabilisatorgemisch aus Calciumchlorid und einem weiteren bekannten organischem Stabilisator in einer Menge von 5 Gew.-%, bezogen auf das in den Zusammensetzungen enthaltende Dichlortrifluorethan, zugesetzt. Es wurden die binären Gemische Nitromethan/Calciumchlorid und 3-Methylbuten-3-ol-1/Calciumchlorid eingesetzt, wobei das Mischungsverhältnis der einzelnen Komponenten zueinander in der jeweiligen genannten Stabilisatormischung 1:1 betrug. Zum Vergleich wurde eine Mischung aus den bekannten organischen Stabilisatorsubstanzen 3-Methylbuten-3-ol-1/Alloocimen, deren Mischungsverhältnis ebenfalls 1:1 betrug, verwendet. Die mit den Stabilisatorgemischen stabilisierten Dichlortrifluorethan enthaltenen Polyolvormischungen wurden, wie bereits beschrieben, mit 140 Gewichtsteilen Diisocyanat MDI 110 verschäumt. Die erhaltenen Polyurethan-Hartschaumstoffe wurden anschließend für 7 bzw. 14 Tage bei 54 °C gelagert. Deren Gehalt an R133a wurde anschließend gaschromatographisch, wie beschrieben, bestimmt.

Tabelle IV zeigt die Ergebnisse, die bei der Stabilisierung von Dichlortrifluorethan in Polyurethan-Schaumstoffen mit binären Stabilisatorgemischen aus Calciumchlorid und jeweils einen weiteren herkömmlichen organischen Stabilisator erreicht werden.

Tabelle IV

| Stabilisierung von Dichlortrifluorethan in Polyurethan-Schaumstoffen mit 5 Gew.-% Stabilisatorgemisch (bezogen auf Dichlortrifluorethan) nach Lagerung bei 54 °C | | | |
|---|---|---|---|
| Bsp. Nr. | Stabilisierung mit | Menge an R133a im Schaumstoff [ppm] nach | |
| | | 7 Tagen | 14 Tagen |
| 18 | Nitromethan/$CaCl_2 \cdot 2H_2O$ | 306 | 549 |
| 19 | 3-Methylbuten-3-ol-1/$CaCl_2 \cdot 2.H_2O$ | 305 | 658 |
| V8 | 3-Methylbuten-3-ol-1/Alloocimen | 348 | 832 |
| V9 | ohne Zusatz | 388 | 853 |

Wie die in Tabelle IV dargestellten Ergebnisse zeigen, wird durch die erfindungsgemäße Stabilisierung von Dichlortrifluorethan durch Calciumchlorid auch in Kombination mit weiteren bereits bekannten Stabilisatorsubstanzen wie Nitromethan oder 3-Methylbuten-3-ol-1 eine gute Stabilisierung von Dichlortrifluorethan in Polyurethan-Schaumstoffen erreicht. Besonders auffällig ist bei diesen Beispielen die verbesserte Langzeitstabilität, die durch die erfindungsgemäße Stabilisierung von Dichlortrifluorethan mit Calciumchlorid in Kombination mit einer herkömmlichen organischen Stabilisatorsubstanz im Vergleich zu einem bekannten Stabilisatorgemisch wie 3-Methylbuten-3-ol-1/Allociman erreicht wird.

**Patentansprüche**

1. Verfahren zur Stabilisierung einer flüssigen Dichlortrifluorethan enthaltenden Zusammensetzung, dadurch gekennzeichnet, daß man eine zur Stabilisierung des Dichlortrifluorethangehaltes wirksame Menge von mindestens einem Lithium- und/oder Erdalkalimetallsalz der Zusammensetzung zusetzt oder mit der Zusammensetzung in Kontakt bringt.

2. Verfahren nach Anspruch 1, dadurch gekennzeichnet, daß die zu stabilisierende Zusammensetzung im wesentlichen Dichlortrifluorethan oder eine Dichlortrifluorethan und gegebenenfalls weitere Treibmittel enthaltende Treibmittelzusammensetzung oder eine Dichlortrifluorethan und gegebenenfalls weitere Kältemittel enthaltende Kältemittelzusammensetzung ist.

3. Verfahren nach Anspruch 1, dadurch gekennzeichnet, daß man die Zusammensetzung mit mindestens einem Lithium- und/oder Erdalkalimetallsalz in Kontakt bringt, indem man die Zusammensetzung durch ein das Lithium- und/oder Erdalkalimetallsalz in fester Form enthaltendes Behältnis leitet.

4. Verfahren nach Anspruch 1, dadurch gekennzeichnet, daß die zu stabilisierende Zusammensetzung eine zur Herstellung von Polyurethan-Schaumstoffen geeignete Polyolvormischung ist, welche ein Gemisch aus monomeren organischen Aminen und Polyolen und/oder Aminogruppen enthaltende Polyole sowie Dichlortrifluorethan und gegebenenfalls weitere zur Polyurethan-Schaumstoffherstellung übliche Zusatzstoffe enthält.

5. Verfahren nach Anspruch 1 bis 4, dadurch gekennzeichnet, daß man als Lithium- und/oder Erdalkalimetallsalz ein Chlorid oder Nitrat einsetzt.

6. Verfahren nach Anspruch 5, dadurch gekennzeichnet, daß man ein Chlorid der Erdalkalimetalle Magnesium und/oder Calcium einsetzt.

7. Verfahren nach Anspruch 1, dadurch gekennzeichnet, daß man den zu stabilisierenden Dichlortrifluorethan enthaltenden Zusammensetzungen das Lithium- und/oder Erdalkalimetallsalz in einer Menge von 0,05 bis 5 Gew.-%, vorzugsweise in einer Menge von 0,5 bis 2 Gew.-%, der in den Zusammensetzungen enthaltenden Dichlortrifluorethanmenge zusetzt.

8. Flüssige Dichlortrifluorethan enthaltende Zusammensetzung, dadurch gekennzeichnet, daß sie eine zur Stabilisierung des Dichlortrifluorethangehaltes wirksame Menge von mindestens einem Lithium- und/ oder Erdalkalimetallsalz enthält.

9. Zusammensetzung nach Anspruch 8, dadurch gekennzeichnet, daß sie im wesentlichen Dichlortrifluorethan oder eine Dichlortrifluorethan und gegebenenfalls weitere Treibmittel enthaltende Treibmittelzusammensetzung oder eine Dichlortrifluorethan und gegebenenfalls weitere Kältemittel enthaltende Kältemittelzusammensetzung ist.

10. Zusammensetzung nach Anspruch 8, dadurch gekennzeichnet, daß sie eine zur Herstellung von Polyurethan-Schaumstoffen geeignete Polyolvormischung ist, welche ein Gemisch aus monomeren organischen Aminen und Polyolen und/oder Aminogruppen enthaltende Polyole sowie Dichlortrifluorethan und gegebenenfalls weitere zur Polyurethan-Schaumstoffherstellung übliche Zusatzstoffe enthält.

11. Zusammensetzung nach Anspruch 8 bis 10, dadurch gekennzeichnet, daß sie als Lithium- und/oder Erdalkalimetallsalz ein Chlorid oder Nitrat enthält.

12. Zusammensetzung nach Anspruch 11, dadurch gekennzeichnet, daß sie ein Chlorid der Erdalkalimetalle Magnesium und/oder Calcium enthält.

13. Zusammensetzung nach Anspruch 8 bis 12, dadurch gekennzeichnet, daß sie das Lithium- und/oder Erdalkalimetallsalz in einer Menge von 0,05 bis 5 Gew.-%, vorzugsweise in einer Menge von 0,5 bis 2 Gew.-%, der in der Zusammensetzung enthaltenden Dichlortrifluorethanmenge enthält.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A | WORLD PATENTS INDEX LATEST<br>Derwent Publications Ltd., London, GB;<br>AN 89-103891<br>& JP-A-1 050 829 (ASAHI GLASS)<br>* Zusammenfassung *<br>--- | | C09K5/04<br>C11D7/50<br>C09K3/30<br>C07C17/42<br>C09K15/02 |
| A | PATENT ABSTRACTS OF JAPAN<br>& JP-A-11 039 539 ( ASAHI GLASS ) 1. Juni 1989<br>* Zusammenfassung *<br>--- | | |
| A | DE-A-2 004 610 (MEDILINE)<br>--- | | |
| P,A | CHEMICAL ABSTRACTS, ,<br>Columbus, Ohio, US;<br>abstract no. 116:10362X,<br>& PL-A-153332 (Hozer et.al.)<br>* Zusammenfassung *<br>--- | | |
| P,A | WO-A-9 113 969 (DU PONT DE NEMOURS)<br><br>----- | | |

**RECHERCHIERTE SACHGEBIETE (Int. Cl.5 )**

C09K
C11D
C07C

Der vorliegende Recherchenbericht wurde für alle Patentansprüche erstellt

| Recherchenort | Abschlußdatum der Recherche | Prüfer |
|---|---|---|
| DEN HAAG | 05 JUNI 1992 | NICOLAS H.J.F. |

EPO FORM 1503 03.82 (P0403)